# EUROPÄISCHE PATENTSCHRIFT

(11) **EP 0 902 006 B1**
(45) Veröffentlichungstag und Bekanntmachung des Hinweises auf die Patenterteilung: **11.05.2005**
(21) Anmeldenummer: 98115429.7
(22) Anmeldetag: 17.08.1998
(51) Int. Cl.: C11C 3/12, C07C 57/12, C07C 51/09, C07C 51/36, C07C 51/43, C07C 51/44, A61K 7/00, A61K 31/23, C11D 1/04, C07C 69/58, C07C 33/025

(54) **Oxidationsstabile Oleine**
Oxidation stable oleins
Oléines stables à l'oxydation

(30) Priorität: 25.08.1997 DE 19736737
(43) Veröffentlichungstag der Anmeldung: 17.03.1999
(73) Patentinhaber: Cognis Deutschland GmbH & Co. KG, 40589 Düsseldorf (DE)
(72) Erfinder: Christoph, Ralf, Dr., 40764 Langenfeld (DE); Petersen, Heinrich, 40591 Düsseldorf (DE); Steinberner, Udo, Dr., 40724 Hilden (DE); Strube, Albert, Dr., 41470 Neuss (DE)

(56) Entgegenhaltungen:
- US-A- 2 457 611
- US-A- 3 860 569
- US-A- 4 179 454

## Beschreibung

### Gebiet der Erfindung

Die vorliegende Erfindung betrifft oxidationsstabile überwiegend ungesättigte Fettsäuren ("Oleine") pflanzlichen Ursprungs, ein Verfahren zu ihrer Herstellung sowie die Verwendung der Oleine als Ausgangsstoffe zur Herstellung oberflächenaktiver Substanzen.

### Stand der Technik

Oleine sind technische Gemische von ungesättigten und gesättigten Fettsäuren, die überwiegend Ölsäure enthalten. Üblicherweise werden Oleine aus den natürlich vorkommenden Fetten und Ölen, insbesondere tierischen Ölen gewonnen. Für die Gewinnung von Olein aus Talg ist aus dem Stand der Technik beispielsweise folgendes Verfahren bekannt: Die Triglyceride werden üblicherweise einer Druckspaltung unterworfen und anschließend die resultierenden Gemische der verschiedenen Fettsäuren beispielsweise mittels einer Umnetztrennung in einen überwiegend gesättigten (Stearin) und einen überwiegend ungesättigten Anteil (Olein) getrennt. Das erhaltene Olein kann direkt oder nach weiterer Aufreinigung durch Destillation oder nach entsprechender Derivatisierung beispielsweise in Schmiermitteln, Kosmetika, Salben, Nahrungsmitteln und/oder Futtermitteln eingesetzt werden. In der US-A-2457611 wird ein Verfahren zur selektiven Hydrierung von tierischen und pflanzlichen Fetten und Ölen beschrieben, wobei Linolsäure in Ölsäure umgewandelt wird, die anschließende Fettsäurespaltung und Trennung der Spaltfettsäuren und destillative Aufarbeitung zu farb- und geruchsstabiler Ölsäure führt. Üblicherweise erfolgt die Herstellung von Olein ausgehend von Talg. Bei Durchführung dieses Verfahrens mit Ausgangsstoffen die einen erhöhten Anteil an mehrfach ungesättigten Fettsäuren enthalten, wie dies insbesondere bei pflanzlichen Ausgangsstoffen der Fall ist, erhält man Oleine mit deutlich schlechteren Eigenschaften insbesondere bezüglich der Oxidationsstabilität. Bislang ist pflanzliches Olein beispielsweise aus Palmöl oder Olivenöl zugänglich. Oleine auf Basis von Olivenöl weisen im allgemeinen unerwünscht hohe Trübungspunkte oberhalb 15 und sogar oberhalb von 20 °C auf. Des weiteren wirkt sich der hohe Anteil an mehrfach ungesättigten Fettsäuren negativ auf die Oxidationsstabilität aus. Die Gewinnung von Olein aus Palmkernöl ist durch die komplexe Aufarbeitung sehr aufwendig und teuer außerdem sind nur begrenzte Mengen auf diesem Wege erhältlich. Die in den letzten Jahren zunehmende Diskussion über BSE im Zusammenhang mit Produkten tierischen Ursprungs führte insbesondere im Bereich der Kosmetik dazu, nach alternativen Produkten, vorzugsweise auf pflanzlicher Basis zu suchen. Der einfache Ersatz von Talg beispielsweise durch Palmöl ist aufgrund der bereits erwähnten Unterschiede bezüglich des Fettsäuregehalts und -verteilung mit Qualitätsproblemen der erhaltenen Oleine verbunden.

Aufgabe der vorliegenden Erfindung war es daher, ein technisch leicht durchzuführendes Verfahren zur Herstellung verbesserter Oleine zu entwickeln, durch das ungesättigte Fettsäuren auf Basis pflanzlicher Rohstoffe auch in großen Mengen zugänglich ist. Die verbesserten Oleine sollten sich insbesondere durch einen Trübungspunkt unterhalb von 11°C, eine hohe Oxidationsstabilität sowie eine gute Farbstabilität auszeichnen.

### Beschreibung der Erfindung

Gegenstand der Erfindung sind oxidationsstabile Oleine mit einer Iodzahl von 60 bis 130, einem Trübungspunkt unterhalb von 11°C, die im wesentlich ungesättigte Fettsäuren enthalten und dabei mehr als 65 Gew.-% Ölsäure, jedoch weniger als 16 Gew.-% Linolsäure aufweisen, die man dadurch erhält, daß man pflanzliche Fette und/oder Öle
(a) gegebenenfalls partiell härtet,
(b) in an sich bekannter Weise in Fettsäure und Glycerin spaltet,
(c) die erhaltenen Spaltfettsäuren gegebenenfalls partiell härtet und ,
(d) die Spaltfettsäuren einer Trennung in im wesentlichen ungesättigte Fettsäuren (Oleine) und im wesentlichen gesättigte Fettsäuren unterwirft und
(e) anschließend das erhaltene Roholein destillativ aufarbeitet,
mit der Maßgabe, daß mindestens eine partielle Härtung im Laufe des Gesamtverfahrens durchgeführt wird.

Überraschenderweise wurde festgestellt, daß sich durch die Kombination von partieller Härtung und Olein/Stearintrennung nach destillativer Aufarbeitung Oleine herstellen lassen, die sich durch niedrige Trübungspunkte auszeichnen. Dies ist insbesondere dann der Fall, wenn man von pflanzlichen Rohstoffen ausgeht, die große Mengen an mehrfach ungesättigten Fettsäuren enthalten. Die üblicherweise bei der partiellen Härtung entstehenden trans-Fettsäuren werden gemäß dem Verfahren der vorliegenden Erfindung im Rahmen der Olein-, Stearintrennung, vorzugsweise mittels Netzmitteltrennung gleichzeitig mit den gesättigten Anteilen abgetrennt. Insbesondere überraschend war, daß die erfindungsgemäß hergestellten Oleine deutlich oxidationsstabiler waren als die aus dem Stand der Technik bekannten Produkte - insbesondere tierischen Ursprungs - obwohl der Linolsäuregehalt bei den erfindungsgemäßen Oleinen höher liegt.

Ein weiterer Gegenstand der Erfindung betrifft ein Verfahren zur Herstellung von oxidationsstabilen Oleinen mit einer Iodzahl von 60 bis 130, einem Trübungspunkt unterhalb von 11 °C, die im wesentlich ungesättigte Fettsäuren enthalten und dabei mehr als 65 Gew.-% Ölsäure, jedoch weniger als 16 Gew.-% Linolsäure aufweisen, welches sich dadurch auszeichnet, daß man pflanzliche Fette und/oder Öle
(a) gegebenenfalls partiell härtet,
(b) in an sich bekannter Weise in Fettsäure und Glycerin spaltet,
(c) die erhaltenen Spaltfettsäuren gegebenenfalls partiell härtet und
(d) die Spaltfettsäuren einer Trennung in im wesentlichen ungesättigte Fettsäuren (Oleine) und im wesentlichen gesättigte Fettsäuren unterwirft und
(e) anschließend das erhaltene Roholein destillativ aufarbeitet,
mit der Maßgabe, daß mindestens eine partielle Härtung im Laufe des Gesamtverfahrens durchgeführt wird.

### Olein

Im Rahmen der vorliegenden Erfindung sind unter Oleinen Mischungen von überwiegend ungesättigten Fettsäuren mit Anteilen gesättigter Fettsäuren zu verstehen. Zur Definition von Oleinen siehe auch **Römpp 9. Aufl., Bd.4, 3106**. Die erfindungsgemäßen Oleine sind pflanzlicher Herkunft, vorzugsweise auf Basis von erucaarmen Rapsöl (das auch als Rapsöl der neuen Züchtung bezeichnet wird) und weisen einen Ölsäuregehalt von mehr als 65, vorzugsweise mehr als 75 und insbesondere mehr als 83 Gew.-% auf. Sie zeichnen sich durch ihre hohe Farb- und Oxidationsstabilität sowie einen geringen Eigengeruch aus. Die erfindungsgemäßen Oleine weisen eine Oxidationsstabilität von mehr als 45, insbesondere mehr als 55 und besonders bevorzugt mehr als 65 Stunden, bestimmt mittels einem Oxidographen in Anlehnung an den Sylvester-Test N.D. von 1941, auf. Diese Eigenschaften lassen sich nur teilweise auf den relativ geringen Gehalt an mehrfach ungesättigten Verbindungen zurückführen, insbesondere einen Linolsäuregehalt von weniger als 16, vorzugsweise unterhalb von 15 und insbesondere kleiner 7 Gew.-%. Gleichzeitig liegt die Iodzahl der erfindungsgemäßen Oleine zwischen 60 und 130 und vorzugsweise zwischen 75 bis 115. Der Trübungspunkt der erfindungsgemäßen Oleine liegt unter 11, vorzugsweise unter 8 und insbesondere unter 5 °C. Weiterhin enthalten die erfindungsgemäßen Oleine nur geringe Anteile gesättigter Fettsäuren, so liegt der Gehalt an Palmitinsäure unter 5, insbesondere unterhalb von 4 und vorzugsweise unterhalb von 3 Gew.-% während der Stearinsäuregehalt weniger als 4, vorzugsweise weniger als 2 und insbesondere weniger als 1 Gew.-% beträgt. Die erfindungsgemäßen Oleine können ausgehend von den vorzugsweise durch Druckspaltung erhaltenen Spaltfettsäuren, oder bevorzugt ausgehend von natürlichen, insbesondere pflanzlichen Fetten und Ölen hergestellt werden.

### Spaltfettsäuren

Als Spaltfettsäuren kommen erfindungsgemäß alle Fettsäuremischungen in Frage, die ausgehend von pflanzlichen Fetten und Ölen durch Fettspaltung erhalten werden können. Es handelt sich dabei um technische Mischungen von Fettsäuren. Unter Fettsäuren sind dabei aliphatische Carbonsäuren der Formel (I) zu verstehen,

**R**^{**1**}**CO-OH (I)**

in der R¹CO für einen aliphatischen, linearen oder verzweigten Acylrest mit 6 bis 22 Kohlenstoffatomen und 0 und/oder 1, 2 oder 3 Doppelbindungen steht. Vorzugsweise handelt es sich um lineare Carbonsäuren, insbesondere pflanzlichen Ursprungs. Typische Beispiele sind Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Elaeostearinsäure, Arachinsäure, Gadoleinsäure, Behensäure und Erucasäure.

### Fettsäureglyceride

Die beim erfindungsgemäßen Verfahren als Ausgangsmaterialien einzusetzenden Fettsäureglyceride können die üblichen natürlichen pflanzlichen Fette oder Öle sein. Hierzu gehören beispielsweise Palmöl, Palmkernöl, Baumwollsaatöl, Kokosöl, Erdnußöl, Olivenöl, Leinöl, Babassuöl, Teeöl, Olivenkernöl, Meadowfoamöl, Chaulmoograöl, Korianderöl, Sojaöl, Ricinusöl, sowie Sonnenblumenöl und Rapsöl der alten und neuen Züchtung. Die Hauptbestandteile dieser Fette und Öle sind Glyceride verschiedener Arten von Fettsäuren, die beträchtliche Mengen an Verunreinigungen wie etwa Aldehydverbindungen, Phospholipidverbindungen und freie Fettsäuren enthalten. Diese Materialien können direkt oder nach vorheriger Aufreinigung eingesetzt werden. Bevorzugt werden pflanzliche Fette und Öle mit einem hohen Ölsäureanteil eingesetzt, wie beispielsweise Palmöl, Sonnenblumenöl der neuen Züchtung d.h. mit hohem Ölsäure- und geringem Linolsäureanteil und insbesondere (erucaarmes) Rapsöl der neuen Züchtung. Neben den reinen natürlich vorkommenden Ölen können jedoch auch Mischungen der verschiedenen Öle untereinander oder Vormischungen, wie sie bei technischen Prozessen anfallen und im Handel erhältlich sind, eingesetzt werden.

### Verfahren

Die erfindungsgemäßen Oleine sind erhältlich aus pflanzlichen Fetten und Ölen, die vorzugsweise auf der Stufe der Triglyceride partiell gehärtet werden und anschließend mittels Fettspaltung in Spaltfettsäure und Glycerin getrennt werden. In einer alternativen Ausführungsform erfolgt die partielle Härtung auf der Stufe der Spaltfettsäure. Anschließend wird das Fettsäuregemisch mittels Olein-/Stearintrennung in einen überwiegend gesättigten und einen überwiegend ungesättigten Anteil getrennt. Der Anteil mit den überwiegend ungesättigten Fettsäuren stellt das Roholein dar, welches noch durch anschließende Destillation aufgearbeitet werden kann.

### Fettspaltung

Die Fettspaltung kann gemäß den aus der Literatur bekannten Verfahren kontinuierlich oder diskontinuierlich durchgeführt werden. Sie kann unter verschiedenen Bedingungen durchgeführt werden. Gemäß dem Twitchell-Verfahren arbeitet man kontinuierlich oder diskontinuierlich unter Atmosphärendruck in Gegenwart von Schwefelsäure oder Alkylarylsulfonsäuren. Daneben ist auch als kontinuierlicher Prozeß das Hoffmann-Verfahren bekannt, bei dem die Umsetzung im Rohrreaktor bei 25 bis 50 bar und 220 bis 260 °C im Gegenstrom erfolgt. Auch Enzyme lassen sich zur Fettspaltung einsetzen, doch großtechnisch wird am häufigsten die Umsetzung mit Wasser unter Druck genutzt. Aus der **DE 3403021 A1** ist ein Verfahren zur Gewinnung von Fettsäuren durch einen zweistufigen Prozeß bekannt. Vorzugsweise wird die Fettspaltung kontinuierlich bei einer Temperatur im Bereich von 210 bis 260 °C durchgeführt.

### Härtung

Wesentlich für die vorliegende Erfindung ist die Durchführung einer partiellen Härtung entweder nach oder bevorzugt vor der Druckspaltung. Unter partieller Härtung im Rahmen der vorliegenden Erfindung ist die Härtung der höher ungesättigten Anteile, insbesondere Linolsäure in einfach ungesättigte Ölsäure zu verstehen. Gleichzeitig erfolgt die Härtung eines gewissen Anteils der Ölsäure zu Stearinsäure, wobei jedoch der Gesamtölsäuregehalt in etwa konstant bleibt. Durch Überführung der höher ungesättigten Anteile, insbesondere der Linolsäure in die einfach ungesättigte Ölsäure ist es nun möglich ausgehend von natürlichen, insbesondere pflanzlichen Rohstoffen Oleine mit einer erhöhten Oxidations- und Farbstabilität zu erhalten. Gleichzeitig wird durch die Erhöhung des Stearinanteils eine schärfere Trennung in Olein- und Stearinanteil beispielsweise im Rahmen des Umnetzverfahrens ermöglicht. Überraschenderweise hat sich jedoch gezeigt, daß der geringe Linolsäuregehalt nicht allein die hohe Oxidationsstabilität bedingt, da auf anderen Wegen erhaltene Oleine, beispielsweise tierischen Ursprungs, mit geringeren Linolsäuregehalten oxidationsempfindlicher sind. Erfindungsgemäß wird die partielle Härtung mit einem üblichen aus dem Stand der Technik für solche Verfahren bekannten Katalysator durchgeführt. Bevorzugt handelt es sich um einen Katalysator auf Basis von Nickel. Die partielle Härtung wird bei einer Temperatur von vorzugsweise 120 bis 180 insbesondere 130 bis 150 °C und einem Wasserstoffdruck von 1 bis 4, insbesondere 2 bis 3 bar durchgeführt. Bevorzugt weisen die Fette und Öle bzw. die Spaltfettsäuren nach der partiellen Härtung Iodzahlen im Bereich von 60 bis 130, insbesondere 75 bis 115 auf.

### Olein-/ Stearintrennung

Zur Trennung der ungesättigten und gesättigten Fettsäuren sind aus dem Stand der Technik verschiedene Verfahren bekannt, die im Rahmen des erfindungsgemäßen Verfahrens angewendet werden können. Die Trennung kann beispielsweise anhand des sogenannten "Alfa-Laval"-Verfahrens erfolgen, wie es in **J.Am.Oil.Chem.Soc., 61, 219 ff (1984)** beschrieben ist, oder mittels Lösungsmitteltrennung, dem sogenannten Emersol-Verfahren, durchgeführt werden. Besonders bevorzugt erfolgt die Trennung in Olein und Stearin-Anteil mittels Umnetzverfahren, welches am Beispiel der Talgfettsäure gut untersucht ist. Bei diesem Verfahren wird das Fettsäuregemisch zunächst abgekühlt, bis die Stearinsäure in der flüssigen Ölsäure auskristallisiert und anschließend eine Netzmittellösung, beispielsweise Alkylsulfat hinzugegeben um die Ölsäure zu emulgieren. Durch folgende Zentrifugation werden Ölsäurephase und Stearin-/Wasserphase getrennt (**Fat Sci.Technol. 89, 237 (1987)**).

### Destillation

Zur Aufreinigung des Roholeins schließt sich vorzugsweise eine Destillation bei einer maximalen Sumpftemperatur von 260 °C und einem Vakuum von 2 bis 10 mbar, insbesondere 3 mbar an. Die erfindungsgemäßen Oleine lassen sich zur Herstellung von Derivaten, insbesondere Oleinestern, - amiden und/oder ungesättigten Alkoholen verwenden, welche über die gleichen hervorragenden Eigenschaften im Bezug auf Farb-, Geruchs- und Oxidationsstabilität verfügen wie die Oleine selber. Weiterhin lassen sich sowohl die Oleine selber als auch deren Derivate gemäß der vorliegenden Erfindung in Wasch- und Reinigungsmitteln sowie kosmetischen und/oder pharmazeutischen Formulierungen einsetzen. Eine bevorzugte Verwendung der Oleine ist deren Hydrierung zur Herstellung von ungesättigten Alkoholen. Insbesondere bevorzugte Ester die aus den erfindungsgemäßen Oleinen hergestellt werden können, sind beispielsweise Sorbitanoleat, Cetyloleat, Oleyloleat, und/oder Decyloleat.

### Beispiele

**Beispiel 1.** Die Rapsölspaltfettsäuren wurden über einem handelsüblichen Nickelkatalysator bei einem Druck von 1 bar, bei 135°C für 30 Minuten angehärtet. Anschließend wurden per Umnetztrennung gemäß der Bedingungen angegeben in **Fat. Sci.Technol. 89 237 (1987)** in Olein-und Stearinanteil getrennt und bei einer Sumpftemperatur von 260°C und 3 mbar das Roholein destilliert. Die Produkteigenschaften sind Tabelle 1 zu entnehmen.

**Vergleichsbeispiel V1.** Rapsölspaltfettsäuren wurden per Umnetztrennung analog Beispiel 1 in Olein- und Stearinanteil getrennt und das Roholein bei einer Sumpftemperatur von 260 °C und einem Druck von 3 mbar destilliert. Die Produkteigenschaften sind Tabelle 1 zu entnehmen.

**Beispiel 2.** Die Anhärtung des Rapsöls erfolgte über einem handelsüblichen Nickelkatalysator bei einem Druck von 1 bar und einer Temperatur von 170°C für 45 Minuten. Anschließend wurde das Öl bei 245 °C für 4h in Fettsäure und Glycerin gespalten. Die Fettsäurefraktion wurde einer Umnetztrennung in Olein- und Stearinanteil gemäß Beispiel 1 unterworfen. Das Roholein wurde bei einer Sumpftemperatur von 260 °C und 3 mbar destilliert. Die Produkteigenschaften sind Tabelle 1 zu entnehmen.

**Beispiel 3.** Die Anhärtung erfolgte im Unterschied zu Beispiel 3 bei einem Druck von 4 bar und einer Temperatur von 140°C für 60 Minuten. Ansonsten wurde das Verfahren analog zu Beispiel 2 durchgeführt. Die Produkteigenschaften sind Tabelle 1 zu entnehmen.

**Vergleichsbeispiel V2.** Neues Sonnenblumenöl wurde gemäß den zuvor beschriebenen Beispielen einer Fettspaltung und Umnetztrennung unterworfen und anschließend destilliert, jedoch nicht angehärtet. Die Produkteigenschaften sind Tabelle 1 zu entnehmen.

**Vergleichsbeispiel V3.** Palmöl wurde - ohne Härtung - einer Fettspaltung, Umnetztrennung und Destillation unterworfen. Die Produkteigenschaften sind Tabelle 1 zu entnehmen.

**Beispiel 4.** Palmöl wurde zunächst über einem Nickelkatalysator bei einem Druck von 2 bis 3 bar und einer Temperatur von 180 °C für 30 Minuten partiell gehärtet. Anschließend wurde wie im Vergleichsbeispiel V3 weiterverfahren. Die Produkteigenschaften sind Tabelle 1 zu entnehmen.

**Beispiel 5.** Palmöl wurde zunächst über einem Nickelkatalysator bei einem Druck von 2 bar und einer Temperatur von 170 °C für 14 Minuten partiell gehärtet. Anschließend wurde wie im Vergleichsbeispiel V3 weiterverfahren. Die Produkteigenschaften sind Tabelle 1 zu entnehmen.

**Beispiel 6.** Herstellung von Olein auf Basis von Talg. Der Talg wird zunächst bei 2 bis 3 bar und einer Temperatur von 180 °C für 30 Minuten partiell gehärtet und dann mittels Fettspaltung, Netzmitteltrennung und Destillation wie zuvor beschrieben weiterverarbeitet. Die Produkteigenschaften sind Tabelle 1 zu entnehmen.

**Vergleichsbeispiel V4.** Dieses Beispiel repräsentiert die derzeit gemäß dem Stand der Technik übliche Verfahrensweise zur Herstellung von Olein auf Basis von Talg. Der Talg wird mittels Fettspaltung, Netzmitteltrennung und Destillation wie zuvor beschrieben weiterverarbeitet. Die Produkteigenschaften sind Tabelle 1 zu entnehmen.

**Tabelle 1**

| **Versuchsergebnisse** | | | | | | | | | | |
|---|---|---|---|---|---|---|---|---|---|---|
| | **1** | **V1** | **2** | **3** | **V2** | **V3** | **4** | **5** | **6** | **V4** |
| ***C-Kettenverteilung [GC-Flächen-%]*** | | | | | | | | | | |
| < C₁₄ | - | - | - | - | - | 0,8 | 0,5 | 0,6 | 0,6 | 0,1 |
| C₁₄ | - | - | - | - | - | 1,1 | 1,6 | 1,2 | 2,6 | 2,0 |
| C₁₅ | - | - | - | - | - | 0,1 | - | - | 0,8 | 0,3 |
| C₁₆ | 2,7 | 3,9 | 1,8 | 2,6 | 3,4 | 4,7 | 5,0 | 4,1 | 3,3 | - |
| C₁₆, einfach ungesättigt | 0,2 | 0,2 | 0,3 | 0,3 | 0,1 | 0,4 | 0,3 | 0,3 | 4,0 | 5,1 |
| C₁₇ | - | - | - | - | - | 0,1 | - | - | 1,2 | 1,3 |
| C₁₈ | 1,2 | 1,3 | 0,3 | 1,0 | 2,0 | 1,1 | 0,8 | 0,9 | 0,7 | 1,0 |
| C₁₈, einfach ungesättigt | 84,1 | 65,6 | 89,8 | 84,4 | 89,1 | 71,9 | 86,8 | 82,1 | 82,2 | 72,6 |
| C₁₈, zweifach ungesättigt | 9,8 | 19,4 | 6,6 | 10,1 | 5,2 | 19,7 | 4,4 | 10,8 | 2,7 | 11,5 |
| C₁₈, dreifach ungesättigt | 0,2 | 7,8 | - | 0,2 | - | 0,4 | - | - | 0,2 | 0,3 |
| C₂₀ | 0,2 | 0,3 | - | 0,3 | 0,2 | 0,2 | 0,1 | 0,1 | 0,1 | 0,5 |
| C₂₀, einfach ungesättigt | 1,3 | 1,3 | 0,9 | 1,0 | 0,2 | 0,3 | 0,2 | 0,2 | 1,6 | 1,2 |
| C₂₂ | 0,1 | 0,1 | - | - | 0,1 | - | - | - | - | 0,3 |
| C₂₂, einfach ungesättigt | 0,2 | 0,1 | 0,1 | 0,1 | - | - | - | - | - | - |
| % trans berechnet auf Ölsäure | 15,5 | 4,0 | 24,1 | 13,5 | 0,9 | 0,5 | 16,1 | 8,6 | 22,9 | 6,6 |
| ***Säurezahl*** | 198 | 198 | 200 | 200 | 200 | 201 | 202 | 200 | 203 | 202 |
| ***Iodzahl*** | 96 | 116 | 95 | 98 | 89 | 101 | 85 | 93 | 81 | 97 |
| ***Trübungspunkt [°C]*** | 7,5 | 3,5 | 6,5 | 4,5 | 10,5 | 6,0 | 8,5 | 5,0 | 5,0 | 3,5 |
| ***Oxidationsstabilität [h]*** | nb | nb | 70 | 70 | 45 | nb | nb | nb | 50 | 15 |

| ***Lovibond-Farbzahl (5 ¼"-Kuvette)*** | | | | | | | | | | |
|---|---|---|---|---|---|---|---|---|---|---|
| - *rot* | 2,1 | nb | 1,1 | 1,0 | 1,5 | 4,6 | 3,5 | 4,6 | 4,0 | 2,2 |
| - *gelb* | 0,1 | | 0,1 | 0,0 | 0,1 | 0,3 | 0,1 | 0,4 | 0,3 | 0,1 |
| nb = nicht bestimmt | | | | | | | | | | |

## Patentansprüche

1. Oxidationsstabile Oleine mit einer Iodzahl von 60 bis 130, einem Trübungspunkt unterhalb von 11°C, die im wesentlich ungesättigte Fettsäuren enthalten und dabei mehr als 65 Gew.-% Ölsäure, jedoch weniger als 16 Gew.-% Linolsäure aufweisen und bestimmt mittels eines Oxidographen in Anlehnung an den Sylvester-Test N.D. von 1941 eine Oxidationsstabilität von mehr als 45 Stunden aufweisen, dadurch erhältlich, daß man pflanzliche Fette und/oder Öle
(a) gegebenenfalls partiell härtet,
(b) in an sich bekannter Weise in Fettsäure und Glycerin spaltet,
(c) die erhaltenen Spaltfettsäuren gegebenenfalls partiell härtet und
(d) die Spaltfettsäuren einer Trennung in im wesentlichen ungesättigte Fettsäuren (Oleine) und im wesentlichen gesättigte Fettsäuren unterwirft und
(e) anschließend das erhaltene Roholein destillativ aufarbeitet,
mit der Maßgabe, daß mindestens eine partielle Härtung im Laufe des Gesamtverfahrens durchgeführt wird.

2. Verfahren zur Herstellung von oxidationsstabilen Oleinen mit einer Iodzahl von 60 bis 130, einem Trübungspunkt unterhalb von 11°C, die im wesentlich ungesättigte Fettsäuren enthalten und dabei mehr als 65 Gew.-% Ölsäure, jedoch weniger als 16 Gew.-% Linolsäure aufweisen und bestimmt mittels eines Oxidographen in Anlehnung an den Sylvester-Test N.D. von 1941 eine Oxidationsstabilität von mehr als 45 Stunden aufweisen, bei dem man pflanzliche Fette und/oder Öle
(a) gegebenenfalls partiell härtet,
(b) in an sich bekannter Weise in Fettsäure und Glycerin spaltet,
(c) die erhaltenen Spaltfettsäuren gegebenenfalls partiell härtet und
(d) die Spaltfettsäuren einer Trennung in im wesentlichen ungesättigte Fettsäuren (Oleine) und im wesentlichen gesättigte Fettsäuren unterwirft und
(e) anschließend das erhaltene Roholein destillativ aufarbeitet,
mit der Maßgabe, daß mindestens eine partielle Härtung im Laufe des Gesamtverfahrens durchgeführt wird.

3. Verfahren nach den Anspruch 2, **dadurch gekennzeichnet, daß** man die partielle Härtung vor der Fettspaltung durchführt.

4. Verfahren nach den Ansprüchen 2 und 3, **dadurch gekennzeichnet, daß** man die partielle Härtung an einem Nickelkatalysator durchführt.

5. Verwendung der Oleine gemäß Anspruch 1 in Wasch- und Reinigungsmitteln sowie kosmetischen und/oder pharmazeutischen Formulierungen.

6. Verwendung der Oleine gemäß Anspruch 1 als Ausgangsstoffe zur Herstellung von ungesättigten Alkoholen (Ocenolen") oder ungesättigten Estern.

## Claims

1. Oxidation-stable oleins with an iodine value of 60 to 130 and a cloud point below 11°C which contain substantially unsaturated fatty acids, of which more than 65% by weight is oleic acid and less than 16% by weight linoleic acid, and have an oxidation stability of more than 45 hours, as determined with an oxidograph by the Sylvester Test N.D. of 1941, obtainable by
(a) optionally partly hydrogenating vegetable fats and/or oils,
(b) hydroylzing them in known manner to fatty acid and glycerol,
(c) optionally partly hydrogenating the fatty acids obtained and
(d) separating them into substantially unsaturated fatty acids (oleins) and substantially saturated fatty acids and
(e) working up the crude olein obtained by distillation,
with the proviso that an at least partial hydrogenation is carried out in the course of the process as a whole.

2. A process for the production of oxidation-stable oleins with an iodine value of 60 to 130 and a cloud point below 11°C which contain substantially unsaturated fatty acids, of which more than 65% by weight is oleic acid and less than 16% by weight linoleic acid, and have an oxidation stability of more than 45 hours, as determined with an oxidograph by the Sylvester Test N.D. of 1941, **characterized in that** it comprises the steps of
(a) optionally partly hydrogenating vegetable fats and/or oils,
(b) hydrolyzing them in known manner to fatty acid and glycerol,
(c) optionally partly hydrogenating the fatty acids obtained and
(d) separating them into substantially unsaturated fatty acids (oleins) and substantially saturated fatty acids and
(e) working up the crude olein obtained by distillation,
with the proviso that an at least partial hydrogenation is carried out in the course of the process as a whole.

3. A process as claimed in claim 2, **characterized in that** the partial hydrogenation is carried out before the hydrolysis step.

4. A process as claimed in claims 2 and 3, **characterized in that** the partial hydrogenation is carried out in the presence of a nickel catalyst.

5. The use of the oleins claimed in claim 1 in detergents and in cosmetic and/or pharmaceutical formulations.

6. The use of the oleins claimed in claim 1 as starting materials for the production of unsaturated alcohols ("Ocenols") or unsaturated esters.

## Revendications

1. Oléines stables à l'oxydation ayant un indice d'iode allant de 60 à 130, un point de trouble en dessous de 11 °C, qui renferment essentiellement des acides gras non saturés et qui possèdent pour cela plus de 65 % en poids d'acide oléique mais moins de 16 % d'acide linoléique et qui possèdent par détermination à l'aide de graphiques d'oxydation en conformité avec le test de Sylvester ND de 1941, une stabilité à l'oxydation de plus de 45 heures, accessibles en ce qu'
a) on durcit éventuellement en partie, des graisses ou des huiles végétales,
b) on coupe d'une manière connue en soi, en acides gras et en glycérol,
c) on durcit éventuellement en partie les acides gras de coupure obtenus, et
d) on soumet les acides gras de coupure à une séparation en acides gras non saturés (oléines) pour l'essentiel et en acides gras essentiellement saturés, et
e) ensuite on purifie les oléines brutes obtenues par distillation ;
avec la précision qu'on effectue au moins un durcissement partiel au cours du processus total.

2. Procédé de préparation d'oléines stables à l'oxydation ayant un indice d'iode de 60 à 130, un point de trouble en dessous de 11°C qui renferment pour l'essentiel des acides gras non saturés, et qui possèdent pour cela plus de 65 % en poids d'acide oléique, mais moins de 16 % en poids d'acide linoléique et qui par détermination à l'aide de graphiques d'oxydation en conformité avec le Test de Sylvester ND de 1941 une stabilité à l'oxydation de plus de 45 heures, dans lequel :
a) on durcit éventuellement partiellement des graisses et/ou des huiles végétales,
b) on coupe d'une manière connue en soi en acides gras et en glycérol,
c) on durcit éventuellement partiellement les acides gras de coupure obtenus,
d) on soumet les acides gras de coupure à une séparation en acides gras essentiellement non saturés (oléines) et en acides gras essentiellement non saturés, et
e) on purifie ensuite l'oléine brute obtenue par distillation,
avec la précision qu'au moins un durcissement partiel est effectué au cours de la totalité du processus.

3. Procédé selon la revendication 2, **caractérisé en ce qu'** on effectue le durcissement partiel avant la coupure des graisses.

4. Procédé selon les revendications 2 et 3, **caractérisé en ce qu'** on effectue le durcissement partiel sur un catalyseur au nickel.

5. Utilisation des oléines selon la revendication 1, dans des produits de lavage et de nettoyage ainsi que dans des formulations cosmétiques et/ou pharmaceutiques.

6. Utilisation des oléines selon la revendication 1, comme produits de départ pour la préparation d'alcools non saturés (Ocenols) ou d'esters non saturés.
